# EUROPEAN PATENT APPLICATION

(11) **EP 4 431 499 A1**
(43) Date of publication of application: **18.09.2024**
(21) Application number: 22913983.7
(22) Date of filing: 30.11.2022
(51) Int. Cl.: C07D 279/20, C07D 279/18, C07C 209/60, C07C 211/55

(54) **METHOD AND SYSTEM FOR PREPARING DIPHENYLAMINE AND PHENOTHIAZINE FROM ANILINE**

(30) Priority: 01.01.2022 CN 202210000026
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: QI, Wenbo, Dalian, Liaoning 116045 (CN); WANG, Zhenyu, Dalian, Liaoning 116045 (CN); LI, Lanpeng, Dalian, Liaoning 116045 (CN); ZHAO, Xiangyu, Dalian, Liaoning 116045 (CN); WANG, Libo, Dalian, Liaoning 116045 (CN); LI, Haomeng, Dalian, Liaoning 116045 (CN); AI, Fubin, Dalian, Liaoning 116045 (CN)
(74) Representative: karo IP
(86) International application number: PCT/CN2022/135596
(87) International publication number: WO 2023/124728

(57) **Abstract**

The present invention relates to the technical field of organic synthesis. Disclosed are a method and system for preparing diphenylamine and phenothiazine from aniline. The method comprises: reacting an aniline raw material; carrying out gas-liquid separation on an obtained reaction mixture containing diphenylamine, and obtaining a first liquid product and a first gas product; separating the first liquid product, and obtaining an aniline reclaimed material and a diphenylamine product; reacting part of the obtained diphenylamine product; carrying out gas-liquid separation on an obtained reaction mixture containing phenothiazine, obtaining a second liquid product and a second gas product, and separating a phenothiazine product from the second liquid product. In the present invention, by coupling a diphenylamine synthesis process and a phenothiazine synthesis process, the heat of the two processes can be coupled, which greatly reduces the energy consumption of the coupled processes. In addition, the output of the two products can be reasonably allocated according to market conditions, and the product purity of diphenylamine can be flexibly adjusted, thereby optimizing the product structure.

## Description

### CROSS-REFERENCE TO RELATED APPLICATIONS

The application claims the benefit of Chinese patent application No. "202210000026.0", filed on January 1, 2022, the content of which is specifically and entirely incorporated herein by reference.

### TECHNICAL FIELD

The present invention relates to the technical field of organic synthesis, particularly to a method and system for preparing diphenylamine and phenothiazine from aniline.

### BACKGROUND ART

Diphenylamine is an important organic raw material that has widespread uses. It is industrially used mainly as an antioxidant for synthetic rubbers, an explosive stabilizer, a fuel and pharmaceutical intermediate, an azo-based dye, a fruit preservative, etc., and can also be used as an analytical reagent for identification of deoxyribonucleic acid (DNA), a colorimetric assay and redox indicator for nitrates, nitrites, chlorates, and magnesium.

Phenothiazine, also known as thiodiphenylamine, is a polymerization inhibitor with excellent performance and is also an intermediate for the synthesis of fine chemicals such as pharmaceuticals and dyes, it per se is an adjuvant for synthetic material (polymerization inhibitor for the production of vinylon), a fruit tree insecticide, and a veterinary anthelmintic.

The Chinese patent application CN105272860A discloses a process for continuously producing diphenylamine with aniline, the technical scheme is realized through the following steps: aniline is adopted as a raw material; a fixed bed catalytic reactor is adopted as a reaction container; and diphenylamine is synthesized under the effect of a catalyst, wherein the reaction is carried out under a temperature range of 200-380°C and a pressure range of 2.5-4.0 MPa.

The Chinese patent application CN105524016A discloses a synthetic method of phenothiazine and/or its derivative thereof, which is characterized in that the synthetic method comprises the following steps: mixing diphenylamine and/or a diphenylamine derivative, sulfur, and a solid acid catalyst; then subjecting the obtained mixture to full reaction in a protective atmosphere; and then carrying out post-treatment to obtain phenothiazine and/or the derivative thereof.

However, the production facility of diphenylamine and the production facility of phenothiazine are two separate industrial devices at present. Diphenylamine is widely applied in a variety of fields, the product specification and required purity of the diphenylamine are different depending on the various downstream products. The current industrial standard merely stipulates a kind of diphenylamine with a purity larger than or equal to 99.6%. However, when diphenylamine is used for producing the polymerization inhibitor phenothiazine, its purity does not need to achieve a high level of 99.6% or more. In the case of the long-term purchase of diphenylamine with a purity larger than or equal to 99.6% to synthesize phenothiazine, not only the raw material costs of the phenothiazine production facility are high, but the diphenylamine production facility will result in a high energy consumption for product refining and a large number of solid wastes, in order to ensure that that all product achieves the purity standard of larger than or equal to 99.6%, thus it causes unnecessary energy and material losses, such a practice is not in compliance with strategic planning of carbon peak and carbon neutrality proposed by the government.

### SUMMARY OF THE INVENTION

The present invention aims to provide a method and system for preparing diphenylamine and phenothiazine from aniline, by coupling the diphenylamine synthesis process with the phenothiazine synthesis process, the invention not only can flexibly adjust the purity of diphenylamine, reduce the energy consumption for refining diphenylamine and decrease the amount of generated solid wastes but also can convey the unreacted diphenylamine after vacuum distillation in the phenothiazine production process to an intermediate component tower of the diphenylamine production process for subjecting to the purification processing, thereby saving the separation step and energy consumption in the phenothiazine production process. Since the diphenylamine with a low purity (larger than or equal to 98.0%) is available for producing phenothiazine, the output of the products diphenylamine and phenothiazine can be reasonably allocated according to the price and market demand conditions, and the product purity of diphenylamine can be flexibly adjusted, thereby optimizing the product structure, and greatly improving the capacity of chemical enterprises for resisting product price fluctuation.

The present invention provides a method for preparing diphenylamine and phenothiazine from aniline, the method comprises the following steps:
(1) performing a reaction on an aniline raw material to obtain a reaction mixture containing diphenylamine;
(2) carrying out gas-liquid separation on the reaction mixture obtained in step (1) to obtain a first liquid product and a first gas product;
(3) subjecting the first liquid product to a separation treatment to obtain an aniline reclaimed material and a diphenylamine product;
(4) performing a reaction on a part of the diphenylamine product obtained in step (3) to obtain a reaction mixture containing phenothiazine;
(5) carrying out gas-liquid separation on the reaction mixture obtained in step (4) to obtain a second liquid product and a second gas product, and separating a phenothiazine product from the second liquid product.

Preferably, the separation treatment process in step (3) is a multi-stage rectification; further preferably, the multi-stage rectification process comprises the following steps:
removing the light component: conveying the first liquid product to a light component removal tower for rectification, and removing the light component from the tower top;
aniline recovery: conveying the tower bottom product of the light component removal tower to an aniline recovery tower for rectification, collecting an aniline reclaimed material from the tower top, and obtaining a diphenylamine-containing material flow from the tower bottom;
removing the intermediate component: feeding the diphenylamine-containing material flow to an intermediate component tower for rectification, removing the intermediate component from the tower top, and obtaining a diphenylamine-rich material flow at the tower bottom;
diphenylamine product rectification: conveying the diphenylamine-rich material flow to a diphenylamine product tower for distillation, receiving the diphenylamine products with different purities from the tower top and side line respectively, and removing the heavy component from the tower bottom.

Preferably, the diphenylamine qualified product is obtained from the tower top of the diphenylamine product tower, the diphenylamine premium grade is extracted from a side line of the diphenylamine product tower, the purity of the diphenylamine qualified product is larger than or equal to 98wt%, the purity of the diphenylamine premium grade is larger than or equal to 99.6wt%; further preferably, the diphenylamine qualified product is used as a reaction raw material of step (4).

Preferably, the aniline reclaimed material obtained in step (3) is recycled as the aniline raw material.

Preferably, the process of separating a phenothiazine product from the second liquid product in step (5) comprises: sequentially subjecting the second liquid product to adsorption purification and vacuum distillation.

Further preferably, the diphenylamine-rich light component obtained from the vacuum distillation process is conveyed to the intermediate component tower for rectification.

Preferably, the first gas product obtained in step (2) and the second gas product obtained in step (5) are mixed for subjecting to the purification treatment.

The present invention also provides a system for preparing diphenylamine and phenothiazine from aniline, the system comprises:
a first reactor for performing reaction on an aniline raw material in the first reactor;
a first gas-liquid separation device for carrying out gas-liquid separation on the reaction mixture obtained in the first reactor to obtain a first liquid product and a first gas product;
a first product separation device for subjecting the first liquid product separated from the first gas-liquid separation device to a separation treatment to obtain an aniline reclaimed material and a diphenylamine product;
a second reactor for performing a reaction on a part of the diphenylamine product obtained from the first product separation device in the second reactor;
a second gas-liquid separation device for carrying out gas-liquid separation on the reaction mixture obtained in the second reactor to obtain a second liquid product and a second gas product; and
a second product separation device for separating a phenothiazine product from the second liquid product separated by the second gas-liquid separation device.

Preferably, the first product separation device is a multi-stage rectification device. Further preferably, the multi-stage rectification device comprises:
a light component removal tower for rectifying the first liquid product separated by the first gas-liquid separation device, and removing the light components from the tower top;
an aniline recovery tower for rectifying the tower bottom product from the light component removal tower, collecting an aniline reclaimed material from the tower top, and obtaining a diphenylamine-containing material flow from the tower bottom;
an intermediate component tower for rectifying the diphenylamine-containing material flow from the aniline recovery tower, removing the intermediate component from the tower top, and obtaining a diphenylamine-rich material flow at the tower bottom; and
a diphenylamine product tower for rectifying the diphenylamine-rich material flow from the intermediate component tower, receiving the diphenylamine products with different purities from the tower top and side line respectively, and removing the heavy component from the tower bottom.

Compared with the prior art, the method and system for preparing diphenylamine and phenothiazine from aniline in the present invention have the following advantages:
1. The existing phenothiazine production devices generally adopt a kettle-type batch operation. The reasons for the lack of a continuous production apparatus may be two aspects: (1) the market demand is stable and the batch operation is sufficient to meet the market demand; and (2) if the continuous operation is adopted, the conversion rate is necessarily reduced, the rectification tower and associated equipment for recovering the diphenylamine must be increased accordingly, which is not economically cost-efficient. In the present invention, the phenothiazine production process is embedded into the diphenylamine synthesis process, which on one hand can achieve the large-scale continuous production of diphenylamine, and optimize the production route of diphenylamine; on the other hand, the fractionation precision of the products in the diphenylamine synthesis process can be appropriately relaxed, the diphenylamine premium grade (with purity larger than or equal to 99.6%) can be used as the product output to the market, and the diphenylamine qualified product can be directly transferred to the phenothiazine production process for further reaction, such an arrangement reduces the energy consumption of the diphenylamine product rectification tower, and meets the requirement for the phenothiazine production process on the diphenylamine raw material, the heat carried by the diphenylamine product also provides a heat source for the reaction of preparing phenothiazine with diphenylamine, which reduces the energy consumption of the phenothiazine production process. Therefore, the coupling processes of the present invention can achieve the coupling of heat from the two processes, thereby greatly reducing the energy consumption of the coupled processes.
2. In the diphenylamine synthesis process, the conversion rate and selectivity are higher in the initial stage of the reaction, so that a small amount of intermediate component is produced, however, the conversion rate and selectivity are significantly decreased in the final stage of the reaction, thus the content of the intermediate components is greatly increased. In order to compromise the separation effect of the products in the later stage of the reaction, the feed position for allowing an entry of the tower bottom material aniline into the intermediate component tower cannot be disposed of at the high side. Therefore, the feeding of the intermediate component tower fluctuates and is difficult to operate in the initial stage of the reaction. In addition, since the phenothiazine preparation process generally adopts the solid acid catalysts, a large amount of intermediate components are produced in the initial stage of the continuous production, due to the high activity of the catalyst, high conversion rate, and the selectivity is not optimal; while in the late stage of reaction, the content of intermediate components will be reduced accordingly because the catalyst activity tends to be stable, the selectivity is gradually increased. In order to compromise the separation effect of the products in the initial stage of the reaction, the feed position for allowing an entry of the tower bottom material aniline into the intermediate component tower should not be too low. Therefore, the intermediate component tower is difficult to operate in the late stage of the reaction. By embedding the phenothiazine preparation process into the diphenylamine synthesis process, the tower bottom material aniline for entry into the intermediate component tower is mixed with the diphenylamine-rich material flow discharged from the top of the vacuum distillation tower in the phenothiazine preparation process, so that the feedstock into the intermediate component tower has a relatively stable composition (i.e., the content of the diphenylamine is relatively stable) throughout the operation cycle, such that the operation fluctuation of the intermediate component tower is alleviated, the operation is stable, and the operation is convenient.
3. The process of the present invention allows for a reasonable adjustment of the product yield of diphenylamine and phenothiazine based on the market price and demand condition of the two products, flexible adjustment of the product purity of diphenylamine, and further optimization of the product structure of the coupled processes, which greatly increases the capacity of chemical enterprises for resisting product price fluctuation.
4. The ammonia gas generated in the diphenylamine production process is an alkaline gas, and the hydrogen sulfide generated in the phenothiazine production process is an acid gas, when the two exhaust gases are simultaneously subjected to water washing, they can be neutralized for each other, thereby reducing the outsourcing amount of the acid and alkali used for the acid absorption and the alkali absorption processes, and decreasing the amount of discharged wastewater.

### BRIEF DESCRIPTION OF THE DRAWINGS

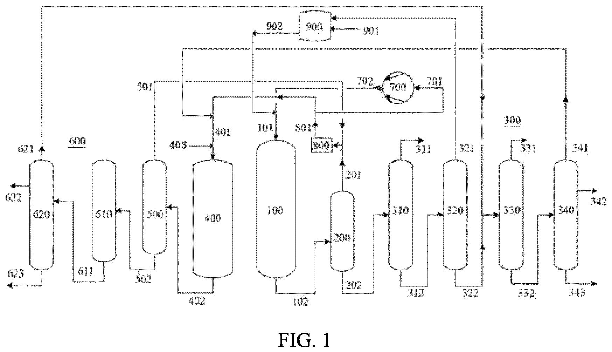

FIG. 1 illustrates a schematic diagram of the technological process for preparing diphenylamine and phenothiazine from aniline according to the invention.

### Description of reference signs

First reactor 100, first feed pipeline 101, first output pipeline 102;
First gas-liquid separation device 200, first gas product transfer pipeline 201, first liquid product transfer pipeline 202;
First product separation device 300, light component removal tower 310, light component transfer pipeline 311, tower bottom product transfer pipeline 312, aniline recovery tower 320, aniline reclaimed material transfer pipeline 321, diphenylamine-containing material flow transfer pipeline 322, intermediate component tower 330, intermediate component transfer pipeline 331, diphenylamine rich material flow transfer pipeline 332, diphenylamine product tower 340, diphenylamine qualified product transfer pipeline 341, diphenylamine premium grade collection pipeline 342, first heavy component output pipeline 343;
Second reactor 400, second feed pipeline 401, second output pipeline 402, sulfur supply pipeline 403;
Second gas-liquid separation device 500, second gas product transfer pipeline 501, second liquid product transfer pipeline 502;
Second product separation device 600, adsorption purification device 610, third output pipeline 611, vacuum distillation apparatus 620, diphenylamine rich material transfer pipeline 621, phenothiazine product collection pipeline 622, second heavy component output pipeline 623;
Gas compressor 700, to-be-compressed gas feed pipeline 701, compressed gas delivery pipeline 702;
Waste gas purification apparatus 800, purified gas output pipeline 801;
Aniline raw material tank 900, fresh aniline pipeline 901, raw material output pipeline 902.

### DESCRIPTION OF THE PREFERRED EMBODIMENT

The specific embodiments of the present invention will be described in detail below with reference to the appended figures. It shall be understood that the specific embodiments described herein merely serve to illustrate and explain the invention, instead of imposing limitation thereto.

The invention provides a method for preparing diphenylamine and phenothiazine from aniline, the method comprises the following steps:
(1) performing a reaction on an aniline raw material to obtain a reaction mixture containing diphenylamine;
(2) carrying out gas-liquid separation on the reaction mixture obtained in step (1) to obtain a first liquid product and a first gas product;
(3) subjecting the first liquid product to a separation treatment to obtain an aniline reclaimed material and a diphenylamine product;
(4) performing a reaction on a part of the diphenylamine product obtained in step (3) to obtain a reaction mixture containing phenothiazine;
(5) carrying out gas-liquid separation on the reaction mixture obtained in step (4) to obtain a second liquid product and a second gas product, and separating a phenothiazine product from the second liquid product.

In step (1), the reaction relates to the synthesis of aniline into diphenylamine, the specific reaction formula is represented by Formula (1), the main product is diphenylamine, and the byproducts are mainly ammonia gas, 2-picoline, 2-ethylaniline, quinoline, indole, acridine, etc.

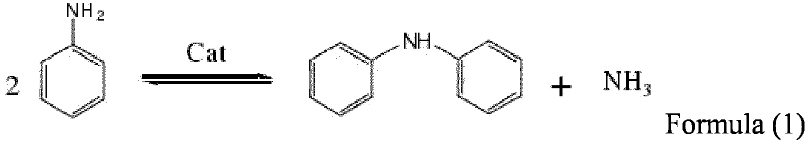

The reaction conditions for preparing diphenylamine with aniline may comprise a reaction temperature within the range of 250-380°C, a reaction pressure within the range of 1-8MPa, and a liquid hourly volume space velocity within the range of 0.05-4h⁻¹. The pressure indicated herein refers to absolute pressure.

The parameter indicators of the aniline raw material for preparing diphenylamine are shown in Table 1, and the parameter indicators of the recyclable aniline raw material are illustrated in Table 2.

**Table 1**

| Items | Indicators, wt% |
|---|---|
| Appearance | Colorless to yellowish transparent liquid, allowing for color darkening in storage |
| Aniline purity | ≥99.80 |
| Nitrobenzene content | ≤0.002 |
| Low boiling component content | ≤0.008 |
| High boiling component content | ≤0.01 |
| Moisture content | ≤0.10 |

**Table 2**

| Items | Indicators, wt% |
|---|---|
| Purity | ≥99.5 |
| Balance | Other ingredients in the reaction fluid composition |

The catalyst used during the reaction process in step (1) is at least one selected from the group consisting of β molecular sieve catalyst, Y molecular sieve catalyst, X molecular sieve catalyst, mordenite, and ZSM-5. In a case of preferably, the catalyst used is β molecular sieve catalyst. The β molecular sieve catalyst may be a conventional choice in the art. In a preferred embodiment, the β molecular sieve catalyst comprises 50-95wt% of β molecular sieve and 5-50wt% of γ-Al₂O₃, based on the total weight of the β molecular sieve catalyst. More preferably, the β molecular sieve catalyst comprises 60-90wt% of β molecular sieve and 10-40wt% of γ-Al₂O₃, based on the total weight of the β molecular sieve catalyst. The β molecular sieve has a SiO₂/Al₂O₃ molar ratio within the range of 20-100, preferably within the range of 28-68.

In the method according to the invention, the reaction in step (1) may be a continuous operation or an intermittent operation, preferably a continuous operation.

When the reaction for preparing diphenylamine with aniline is a continuous operation, the reaction conditions of the continuous operation may comprise a reaction pressure within the range of 1-8MPa, preferably 2-6MPa; a reaction temperature within the range of 250-380°C, preferably 280-360°C; and a liquid hourly volume space velocity within the range of 0.05-1h⁻¹, preferably 0.1-0.5h⁻¹.

In the method of the invention, the reaction in step (1) is preferably performed in the presence of a protective gas, which is preferably nitrogen gas and/or hydrogen gas.

When a protective gas is introduced during the reaction process in step (1), a volume ratio of the protective gas to the aniline raw material may be within the range of 10-1,000:1, preferably 50-500:1.

When a protective gas is introduced during the reaction process in step (1), the method according to the present invention may further comprise: subjecting the first gas product obtained after gas-liquid separation in step (2) to a purification treatment (e.g., acid washing), and then recycling the obtained gas after the purification treatment as a protective gas source.

In the method of the present invention, the gas-liquid separation process of step (2) can be carried out in a gas-liquid separation device (e.g., a gas-liquid separation tower) that is conventional in the art, the first liquid product is extracted from the tower bottom, and the first gas product is extracted from the tower top. The first liquid product comprises unreacted aniline, diphenylamine, and other by-products. The first gas product mainly comprises ammonia gas and a protective gas.

In the method of the invention, it is preferred that the separation treatment process in step (3) is a multi-stage rectification. It is further preferred that the multi-stage rectification process comprises the following steps:
removing the light component: conveying the first liquid product to a light component removal tower for rectification, and removing the light component from the tower top;
aniline recovery: conveying the tower bottom product of the light component removal tower to an aniline recovery tower for rectification, collecting an aniline reclaimed material from the tower top, and obtaining a diphenylamine-containing material flow from the tower bottom;
removing the intermediate component: feeding the diphenylamine-containing material flow to an intermediate component tower for rectification, removing the intermediate component from the tower top, and obtaining a diphenylamine-rich material flow at the tower bottom;
diphenylamine product rectification: conveying the diphenylamine-rich material flow to a diphenylamine product tower for distillation, receiving the diphenylamine products with different purities from the tower top and side line respectively, and removing the heavy component from the tower bottom.

In the rectification process of the diphenylamine product, the diphenylamine qualified product is obtained from the tower top of the diphenylamine product tower, the diphenylamine premium grade is extracted from a side line of the diphenylamine product tower, the purity of the diphenylamine qualified product is larger than or equal to 98wt%, the purity of the diphenylamine premium grade is larger than or equal to 99.6wt%.

In one specific embodiment, the diphenylamine qualified product is used as a reaction raw material of step (4). If necessary, the diphenylamine premium grade may also be used as at least part of the reaction raw material of step (4).

In a preferred embodiment of the present invention, the method may further comprise: recycling the aniline reclaimed material obtained in step (3) as the aniline raw material. The specific operating process is as follows: the aniline reclaimed material collected from the tower top of the aniline recovery tower is conveyed to an aniline raw material tank for supplying the aniline raw material and is used together with the fresh aniline raw material as the reaction raw material for the reaction process in step (1).

The reaction in step (4) relates to the synthesis of diphenylamine into phenothiazine, the reaction is performed in the presence of a sulfur-containing substance, which is at least one selected from the group consisting of sulfur, sodium sulfide, carbon disulfide, and sulfur dioxide. In a preferred circumstance, the sulfur-containing substance is sulfur.

When the sulfur-containing substance used in the reaction of step (4) is sulfur, the specific reaction formula is represented by Formula (2), the main product is phenothiazine, and the by-products are thiol, thioether, and the like which have a large molecular weight, and comprise diphenylamine or phenothiazine as a substituent.

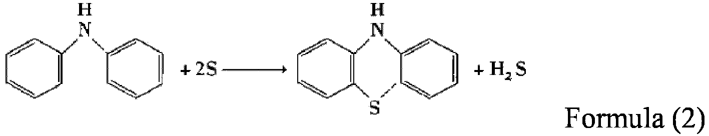

The reaction conditions for the preparation of phenothiazine with diphenylamine may comprise a reaction temperature within the range of 130-250°C and pressure within the range of 5-101kPa in the presence of a catalyst (e.g., iodine tablet).

The parameter indicators of the aniline raw material for preparing diphenylamine are shown in Table 3.

**Table 3**

| Items | Indicators, wt% |
|---|---|
| Appearance | White, light gray, or yellowish tablet |
| Diphenylamine purity | ≥98.0 |
| Impurities | ≤0.12 |
| Moisture content | ≤0.03 |

The catalyst used during the reaction process in step (4) may be a catalyst conventionally used in the art for the synthesis of phenothiazine with diphenylamine. In a more preferred embodiment, the catalyst is at least one selected from the group consisting of an iodine tablet, anhydrous aluminum trichloride, and a solid acid catalyst, and more preferably a solid acid catalyst.

In the method according to the present invention, the reaction in step (4) may be a continuous operation or an intermittent operation. The reaction conditions of the continuous operation in step (4) comprise a reaction pressure within the range of 5-101kPa, preferably 50-101kPa; a reaction temperature within the range of 130-250°C, preferably 160-220°C; and a liquid hourly volume space velocity within the range of 0.1-2h⁻¹, preferably 0.1-0.5h⁻¹. The reaction conditions of the intermittent operation in step (4) comprise a reaction pressure within the range of 5-101kPa, preferably 50-101kPa; a reaction temperature within the range of 130-250°C, preferably 160-220°C; and a reaction time within the range of 2-10h, preferably 4-8h.

In the method of the invention, the reaction in step (4) is performed in the presence of a protective gas, which is preferably nitrogen gas and/or hydrogen gas.

When a protective gas is introduced during the reaction process in step (4), the volume ratio of the protective gas to the aniline raw material is within the range of 50-500:1, preferably 10-100:1.

When a protective gas is introduced during the reaction process in step (4), the method according to the present invention may further comprise: subjecting the second gas product obtained after gas-liquid separation in step (5) to a purification treatment (e.g., alkali washing), and then recycling the obtained gas after the purification treatment as a protective gas source.

In the method of the present invention, the ammonia gas generated in the diphenylamine production process is an alkaline gas, and the hydrogen sulfide generated in the phenothiazine production process is an acid gas when the two exhaust gases are simultaneously subjected to water washing, the ammonia gas and hydrogen sulfide may be neutralized for each other, thereby reducing the outsourcing amount of the acid and alkali used for the acid absorption and the alkali absorption processes, and decreasing the amount of discharged wastewater. Therefore, in a preferred embodiment, the first gas product obtained in step (2) and the second gas product obtained in step (5) are mixed for subjecting to the purification treatment (e.g. water washing).

When a protective gas is introduced into the reaction system of step (1) and/or step (4), it is further preferred that the first gas product obtained in step (2) and the second gas product obtained in step (5) are mixed for subjecting to the purification treatment, the obtained gas after the purification treatment is then recycled as a protective gas source; and if necessary, the gas after the purification treatment is pressurized to a suitable pressure by a gas compressor and subsequently recycled.

In the method of the present invention, the gas-liquid separation process of step (5) can be implemented in a conventional gas-liquid separation device (e.g., a gas-liquid separation tower) in the art, the second liquid product is extracted from the tower bottom, and the second gas product is extracted from the tower top. The second liquid product comprises unreacted diphenylamine, phenothiazine, and other by-products. The second gas product mainly comprises hydrogen sulfide and a protective gas.

In the method of the present invention, the process of separating a phenothiazine product from the second liquid product in step (5) preferably comprises: sequentially subjecting the second liquid product to adsorption purification and vacuum distillation. Further preferably, the method for preparing diphenylamine and phenothiazine from aniline in the present invention may also comprise: conveying the diphenylamine-rich light component obtained from the vacuum distillation process to the intermediate component tower for rectification, thereby enabling recovery and recycling of the unreacted diphenylamine.

In the present invention, the purpose of adsorption purification is subjecting the second liquid product obtained during gas-liquid separation to an adsorption purification treatment, in particular adsorption decoloration. The adsorbent packed in the adsorption purification device may be a conventional adsorbent or adsorbent filler in the art, preferably activated carbon. The adsorption purification device in an intermittent operation is an adsorption kettle, and the adsorption purification device in a continuous operation may comprise an adsorption purification pot or an adsorption purification tower. The adsorption purification device typically employs an A/B pot or A/B tower for switching operation, i.e., one tower is used for purification while another tower performs regeneration of the adsorbent.

In the present invention, the vacuum distillation may be carried out according to conventional methods in the art. In a specific embodiment, the vacuum distillation process may be performed in a distillation still.

As shown in FIG. 1, the system for preparing diphenylamine and phenothiazine from aniline in the present invention comprises:
a first reactor 100 for performing reaction on an aniline raw material in the first reactor 100;
a first gas-liquid separation device 200 for carrying out gas-liquid separation on the reaction mixture obtained in the first reactor 100 to obtain a first liquid product and a first gas product;
a first product separation device 300 for subjecting the first liquid product separated from the first gas-liquid separation device 200 to a separation treatment to obtain an aniline reclaimed material and a diphenylamine product;
a second reactor 400 for performing a reaction on a part of the diphenylamine product obtained from the first product separation device 300 in the second reactor 400;
a second gas-liquid separation device 500 for carrying out gas-liquid separation on the reaction mixture obtained in the second reactor 400 to obtain a second liquid product and a second gas product; and
a second product separation device 600 for separating a phenothiazine product from the second liquid product separated by the second gas-liquid separation device 500.

In the system of the present invention, the first product separation device 300 is a multi-stage rectification device. In a more preferred embodiment, the multi-stage rectification device comprises:
a light component removal tower 310 for rectifying the first liquid product separated by the first gas-liquid separation device 200, and removing the light components from the tower top;
an aniline recovery tower 320 for rectifying the tower bottom product from the light component removal tower 310, collecting an aniline reclaimed material from the tower top, and obtaining a diphenylamine-containing material flow from the tower bottom;
an intermediate component tower 330 for rectifying the diphenylamine-containing material flow from the aniline recovery tower 320, removing the intermediate component from the tower top, and obtaining a diphenylamine-rich material flow at the tower bottom; and
a diphenylamine product tower 340 for rectifying the diphenylamine-rich material flow from the intermediate component tower 330, receiving the diphenylamine products with different purities from the tower top and side line respectively, and removing the heavy component from the tower bottom.

Further preferably, a diphenylamine qualified product transfer pipeline 341 for outputting the diphenylamine qualified product is provided at the tower top of the diphenylamine product tower 340; a diphenylamine premium grade collection pipeline 342 for collecting diphenylamine premium grade is provided at the side of the diphenylamine product tower 340; the diphenylamine qualified product transfer pipeline 341 is in communication with the diphenylamine raw material feed inlet of the second reactor 400.

Further preferably, the second product separation device 600 comprises:
an adsorption purification device 610 for performing adsorption purification on a second liquid product separated by the second gas-liquid separation device 500; and
a vacuum distillation apparatus 620 for performing vacuum distillation on the liquid product treated by the adsorption purification device 610 to obtain the diphenylamine-rich light component and the phenothiazine product.

Further preferably, a diphenylamine-rich material transfer pipeline 621 for outputting the diphenylamine-rich light component is disposed at the top of the vacuum distillation apparatus 620, the diphenylamine-rich material transfer pipeline 621 is in communication with the diphenylamine-containing material flow feed inlet of the intermediate component tower 330.

Further preferably, the system further comprises a waste gas purification apparatus 800, which is in communication with the first gas product transfer pipeline 201 disposed at the top of the first gas-liquid separation device 200 and the second gas product transfer pipeline 501 disposed at the top of the second gas-liquid separation device 500.

Still further preferably, the gas output end of the waste gas purification apparatus 800 is provided with a purified gas output pipeline 801; the purified gas output pipeline 801 may be in communication with a gas inlet of the second reactor 400 for conveying the purified and treated gas (mainly consisting of protective gas, such as nitrogen gas and/or hydrogen gas) to the second reactor 400 as a protective gas source, or the purified gas output pipeline 801 may be in communication with the to-be-compressed gas feed pipeline 701 of the gas compressor 700, the purified and treated gas is pressurized to the reaction pressure and transported to the first reactor 100 via the compressed gas delivery pipeline 702.

Still further preferably, the top of the aniline recovery tower 320 is provided with an aniline reclaimed material transfer pipeline 321, which is in communication with an aniline raw material tank 900 for conveying the aniline reclaimed material collected from the aniline recovery tower 320 to the aniline raw material tank 900 for recycling. The aniline raw material tank 900 is also provided with a fresh aniline pipeline 901 for feeding fresh aniline raw material into the aniline raw material tank 900. The raw material output pipeline 902 of the aniline raw material tank 900 is in communication with the feed pipeline 101 of the first reactor 100.

In one specific embodiment, as shown in FIG. 1, the system for preparing diphenylamine and phenothiazine from aniline comprises a first reactor 100, a first gas-liquid separation device 200, a multi-stage rectification device, a second reactor 400, a second gas-liquid separation device 500, an adsorption purification device 610, and a vacuum distillation apparatus 620, wherein the multi-stage rectification device comprises a light component removal tower 310, an aniline recovery tower 320, an intermediate component tower 330, and a diphenylamine product tower 340; an aniline raw material is fed into the first reactor 100 via a first feed pipeline 101, a reaction mixture is conveyed to the first gas-liquid separation device 200 via a first output pipeline 102 for gas-liquid separation, a first gas product obtained after the gas-liquid separation is fed into a waste gas purification apparatus 800 via a first gas product transfer pipeline 201 for purification treatment, the obtained first liquid product is fed into the light component removal tower 310 via a first liquid transfer product pipeline 202 for rectification, the obtained light component is removed via a light component transfer pipeline 311 at the tower top, the tower bottom product is fed into the aniline recovery tower 320 via a tower bottom product transfer pipeline 312 for rectification, an aniline reclaimed material collected from the tower top is conveyed to the aniline raw material tank 900 via an aniline reclaimed material transfer pipeline 321, a diphenylamine-containing material flow at the tower bottom is transported via a diphenylamine-containing material flow transfer pipeline 322 to the intermediate component tower 330 for rectification, the obtained intermediate component is removed via an intermediate component transfer pipeline 331 at the tower top; a diphenylamine rich material flow at the tower bottom is fed into the diphenylamine product tower 340 via a diphenylamine rich material flow transfer pipeline 332 for rectification, the heavy component at the tower bottom is removed via a first heavy component output pipeline 343; a diphenylamine premium grade collected from a side line is transported via a diphenylamine premium grade collection pipeline 342, a diphenylamine qualified product at the tower top is fed via a diphenylamine qualified product transfer pipeline 341 to the second feed pipeline 401 of the second reactor 400 and conveyed to the second reactor 400; in addition, a reactant sulfur is fed via a sulfur supply pipeline 403 to the second reactor 400 for reaction, the obtained reaction mixture is transported to the second gas-liquid separation device 500 via a second output pipeline 402 for gas-liquid separation, a second gas product obtained after the gas-liquid separation is conveyed to the waste gas purification apparatus 800 via a second gas product transfer pipeline 501 for subjecting to the purification treatment, the obtained second liquid product is fed to the adsorption purification device 610 via a second liquid transfer pipeline 502 for adsorption purification; a material flow after the adsorption purification is fed into a vacuum distillation apparatus 620 via a third output pipeline 611 for vacuum distillation; a diphenylamine rich light component collected from the tower top is conveyed via a diphenylamine rich transfer pipeline 621 to the intermediate component tower 330 for recycling; a phenothiazine product collected from the side line is output via a diphenylamine collection pipeline 622, a heavy component at the tower bottom is removed via a second heavy component output pipeline 623.

In the system of the invention, it is preferred that the various pipelines are installed with one or more valves as required, such as a back pressure valve, shut-off valve, or check valve. The arrangement of a back pressure valve, shut-off valve, and check valve is well-known among those skilled in the art, the content will not be repeatedly described herein.

The method and system for preparing diphenylamine and phenothiazine from aniline according to the present invention will be described below with reference to examples. The examples are implemented under the premise of the technical scheme of the invention, and provide detailed embodiments and specific operation procedures, but the protection scope of the invention is not limited to the examples described below.

Unless otherwise specified, the test methods in the examples below were all conventional methods in the art. Unless otherwise indicated, the experimental materials used in the examples below were commercially available from the biochemical reagent stores.

The specification of aniline raw material used in the following Examples and Comparative Examples was shown in Table 1, which complied with the China National Standard GB/T 2961-2014.

The purity of nitrogen gas used herein was larger than 99.9v%.

The β molecular sieve catalyst used in the continuous synthesis of diphenylamine with aniline had a product brand DA-30, wherein the catalyst had the β molecular sieve content of 73.3wt% (with a SiO₂/Al₂O₃ molar ratio of 30) and the γ-Al₂O₃ content of 26.7wt%. Its physical properties were shown in Table 4.

**Table 4**

| Items | Indicators |
|---|---|
| Appearance | Bar-type |
| Bar diameter, mm | 1.0∼3.0 |
| Length, mm | 2∼10 |
| Specific surface area, m²·g⁻¹ | 400∼600 |
| Specific pore volume, mL·g⁻¹ | 0.3∼0.6 |
| Edgewise compressive strength, N·mm⁻¹ | 5 |
| Pile ratio, g·mL⁻¹ | 0.6∼0.8 |

The catalysts used in the synthesis of phenothiazine with diphenylamine were the conventional solid phosphoric acid catalysts.

The sulfur used in the synthesis of phenothiazine with diphenylamine was the industrial first-grade product.

### Comparative Example 1

In Comparative Example 1, the device for producing diphenylamine with aniline and the device for producing phenothiazine with diphenylamine were two separate devices, which were not coupled.

Producing diphenylamine with aniline: the aniline and nitrogen gas were fed from the bottom of the reactor, and passed through a fixed bed reactor. The reactor was packed with β molecular sieve catalyst. The process conditions and reaction results were illustrated in Table 5.

Producing phenothiazine with diphenylamine: diphenylamine and nitrogen gas were fed into a fixed bed reactor for performing the contact reaction with a solid acid catalyst. The process conditions and reaction results were listed in Table 5.

**Table 5: Reaction conditions and results of Comparative Example 1**

| | Producing diphenylamine with aniline | | | Producing phenothiazine with diphenylamine | |
|---|---|---|---|---|---|
| Run time/day | 30 | 120 | Run time/day | 30 | 120 |
| Reaction pressure/MPa | 3.0 | 3.0 | Molar ratio of diphenylamine to sulfur | 1 | 1 |
| Hourly volumetric space velocity/h⁻¹ | 0.2 | 0.2 | Reaction pressure/kPa | 101 | 101 |
| Reaction temperature/°C | 305 | 355 | Liquid hourly volume space velocity of diphenylamine/h⁻¹ | 0.2 | 0.2 |
| Volume ratio of nitrogen gas to aniline | 150 | 150 | Reaction temperature/°C | 180 | 191 |
| Single-pass conversion rate of aniline, mol% | 25.4 | 19.8 | Volume ratio of nitrogen gas to diphenylamine | 150 | 150 |
| Diphenylamine selectivity, mol% | 98.6 | 96.0 | Single-pass conversion rate of diphenylamine, mol% | 80.5 | 78.2 |
| | | | Single-pass conversion rate of sulfur, mol% | 100 | 100 |
| | | | Phenothiazine selectivity, mol% | 91.2 | 92.5 |

### Examples 1-4

The operation process of Examples 1-4 was as follows:

### (1) Continuous production of diphenylamine with aniline

As shown in FIG. 1, in the presence of the β molecular sieve catalyst, nitrogen gas was passed through a nitrogen gas compressor, the compressed nitrogen gas and the raw material aniline were fed into a first reactor 100 via a respective pipeline, the nitrogen gas and aniline carried out reaction and then discharged from the first reactor 100, the reactants were cooled and fed into a first gas-liquid separator for subjecting to the gas-liquid separation. The separated ammonia-containing nitrogen gas was subjected to the purification treatment and returned to the nitrogen gas compressor, the separated liquid product was first fed into a light component removal tower 310, wherein the light component such as 2-picoline was evaporated from the tower top, the kettle liquid was conveyed to an aniline recovery tower 320. In the aniline recovery tower 320, excess aniline was evaporated from the tower top and then recycled to the reaction system, the kettle liquid was conveyed to an intermediate component tower 330. In the intermediate component tower 330, ethyl aniline or other intermediate component were evaporated from the tower top, the kettle liquid was transported to a diphenylamine product tower 340, and the diphenylamine qualified product (with purity larger than equal to 98wt%) was extracted from the tower top, the diphenylamine premium grade (with purity larger than equal to 99.6wt%) was extracted from the side line, and the heavy component was retained in the kettle liquid.

### (2) Production of phenothiazine with diphenylamine

As shown in FIG. 1, in the presence of a solid phosphoric acid catalyst, the diphenylamine qualified product and nitrogen gas were fed into a second reactor 400 (a fixed bed reactor) via the respective pipeline and carried out a reaction, the reaction mixture following the reaction was cooled and conveyed to a second gas-liquid separator for subjecting to the gas-liquid separation. The separated nitrogen gas containing hydrogen sulfide was subjected to a waste gas purification treatment and then recycled, the separated liquid product was conveyed to an adsorption purification tower. The decolorization treatment was performed in the adsorption purification tower, the treated liquid product was then fed into a vacuum distillation tower. In the vacuum distillation tower, the unreacted diphenylamine and the generated intermediate components were evaporated from the tower top, the product phenothiazine was extracted from the side line, and the heavy component was retained in the kettle liquid.

In Example 1, the unreacted diphenylamine evaporated from the vacuum distillation tower during the process of producing phenothiazine with diphenylamine was treated and then returned to the second reactor 400, the process conditions and reaction results were illustrated in Table 6;

In Example 2, the unreacted diphenylamine evaporated from the vacuum distillation tower during the process of producing phenothiazine with diphenylamine was conveyed to an intermediate component tower 330 in the synthetic process of diphenylamine with aniline for carrying out the refining and recovery of diphenylamine, the process conditions and reaction results were listed in Table 6.

In Examples 3 and 4, the unreacted diphenylamine evaporated from the vacuum distillation tower during the process of producing phenothiazine with diphenylamine was conveyed to an intermediate component tower 330 in the synthetic process of diphenylamine with aniline for carrying out the refining and recovery of diphenylamine, the process conditions and reaction results were listed in Table 7.

**Table 6: Reaction conditions and results of Examples 1 and 2**

| Examples | | Example 1 | | | Example 2 | | |
|---|---|---|---|---|---|---|---|
| Run time/day | | 30 | 80 | 120 | 30 | 80 | 120 |
| Diphenylamine synthesis | Reaction pressure/MPa | 3.0 | 3.0 | 3.0 | 3.0 | 3.0 | 3.0 |
| | Hourly volumetric space velocity/h⁻¹ | 0.2 | 0.2 | 0.2 | 0.2 | 0.2 | 0.2 |
| | Reaction temperature/°C | 305 | 322 | 355 | 305 | 321 | 356 |
| | Volume ratio of nitrogen gas to diphenylamine | 150 | 150 | 150 | 150 | 150 | 150 |
| | Single-pass conversion rate of aniline, mol% | 25.6 | 23.1 | 20.2 | 25.7 | 23.3 | 20.4 |
| | Diphenylamine selectivity, mol% | 98.5 | 97.5 | 96.2 | 98.4 | 97.6 | 96.5 |
| | Circulation ratio* | 0 | 0 | 0 | 0.2 | 0.2 | 0.2 |
| | Feedstock composition of the intermediate component tower, wt% | | | | | | |
| | Aniline, pyridine, and derivatives thereof | 2.22 | 2.39 | 2.5 | 1.89 | 1.99 | 2.1 |
| | Diphenylamine | 95.58 | 95.3 | 95.05 | 81.2 | 80.68 | 79.45 |
| | Thiols and thioethers with substituent diphenylamine | 0 | 0 | 0 | 0.32 | 0.24 | 0.21 |
| | Thiols and thioethers with substituent phenothiazine | 0 | 0 | 0 | 0.18 | 0.16 | 0.12 |
| | Diphenylamine in phenothiazine device | 0 | 0 | 0 | 14.54 | 15.01 | 16.08 |
| | Total amount of diphenylamine | 95.58 | 95.3 | 95.05 | 95.74 | 95.69 | 95.53 |
| Phenothiazine synthesis | The molar ratio of diphenylamine to sulfur | 1 | 1 | 1 | 1 | 1 | 1 |
| | Reaction pressure /kPa | 101 | 101 | 101 | 101 | 101 | 101 |
| | Liquid hourly volume | 0.2 | 0.2 | 0.2 | 0.2 | 0.2 | 0.2 |
| | space velocity of diphenylamine/h⁻¹ | | | | | | |
| | Reaction temperature/°C | 180 | 185 | 191 | 180 | 184 | 190 |
| | Volume ratio of nitrogen gas to diphenylamine | 100 | 100 | 100 | 100 | 100 | 100 |
| | Single-pass conversion rate of sulfur, mol% | 100 | 100 | 100 | 100 | 100 | 100 |
| | Single-pass conversion rate of diphenylamine, mol% | 80.6 | 79.2 | 78.3 | 80.7 | 79.5 | 78.1 |
| | Phenothiazine selectivity, mol% | 91.3 | 93.3 | 92.6 | 91.2 | 93.1 | 92.8 |

| | | | | | | | |
|---|---|---|---|---|---|---|---|
| *: Circulation ratio refers to the mass ratio of the unreacted diphenylamine to the tower bottom material of the aniline recovery tower during the phenothiazine preparation process. | | | | | | | |

**Table 7: Reaction conditions and results of Examples 3 and 4**

| Examples | | Example 3 | | Example 4 | |
|---|---|---|---|---|---|
| Run time/day | | 30 | 120 | 30 | 120 |
| Diphenylamine synthesis | Reaction pressure/MPa | 2.0 | 2.0 | 4.0 | 4.0 |
| | Hourly volumetric space velocity/h⁻¹ | 0.1 | 0.1 | 0.3 | 0.3 |
| | Reaction temperature/°C | 302 | 353 | 307 | 352 |
| | Volume ratio of nitrogen gas to diphenylamine | 100 | 100 | - | - |
| | Single-pass conversion rate of aniline, mol% | 27.6 | 21.3 | 20.5 | 18.6 |
| | Diphenylamine selectivity, mol% | 97.2 | 95.4 | 97.1 | 93.8 |
| | Circulation ratio * | 0.2 | 0.2 | 0.2 | 0.2 |
| | Feedstock composition of the intermediate component tower, wt% | | | | |
| | Aniline, pyridine, and derivatives thereof | 2.12 | 2.24 | 1.97 | 2.22 |
| | Diphenylamine | 80.94 | 78.95 | 79.08 | 78.45 |
| | Thiols and thioethers with substituent diphenylamine | 0.38 | 0.23 | 0.34 | 0.26 |
| | Thiols and thioethers with substituent phenothiazine | 0.21 | 0.14 | 0.19 | 0.15 |
| | Diphenylamine in phenothiazine device | 14.36 | 16.29 | 16.54 | 16.87 |
| | Total amount of diphenylamine | 95.3 | 95.24 | 95.62 | 95.32 |
| Phenothiazine synthesis | The molar ratio of diphenylamine to sulfur | 1 | 1 | 1 | 1 |
| | Reaction pressure /kPa | 101 | 101 | 101 | 101 |
| | Liquid hourly volume space velocity of diphenylamine/h⁻¹ | 0.1 | 0.1 | 0.3 | 0.3 |
| | Reaction temperature/°C | 188 | 195 | 186 | 194 |
| | Volume ratio of nitrogen gas to diphenylamine | 150 | 150 | - | - |
| | Single-pass conversion rate of sulfur, mol% | 100 | 100 | 100 | 100 |
| | Single-pass conversion rate of diphenylamine, mol% | 82.6 | 79.3 | 75.4 | 73.1 |
| | Phenothiazine selectivity, mol% | 89.4 | 90.1 | 88.7 | 89.2 |

| | | | | | |
|---|---|---|---|---|---|
| *: Circulation ratio refers to the mass ratio of the unreacted diphenylamine to the tower bottom material of the aniline recovery tower during the phenothiazine preparation process. | | | | | |

As can be seen from the result data of Examples 1 to 4, the phenothiazine production process uses the diphenylamine qualified product (with purity larger than or equal to 98.0wt%) generated during the diphenylamine synthesis process as the diphenylamine raw material, without affecting the reaction; moreover, the unreacted diphenylamine during the phenothiazine production process is fed into an intermediate component tower during the diphenylamine production process for the recovery of diphenylamine, which enables the continuous production, simplifies the operation and greatly improves the reaction efficiency; in addition, as can be seen from the total amount data of diphenylamine in the intermediate component tower, the percentages of the total amount of diphenylamine treated by the intermediate component tower of Example 1 are as follows: 95.58 wt% after running 30 days, 95.30 wt% after 80 days, and 95.05 wt% after 120 days; the percentages of the total amount of diphenylamine treated by the intermediate component tower of Example 2 are as follows: 95.74 wt% after running 30 days, 95.69 wt% after 80 days, and 95.53 wt% after 120 days; although it appears that the variation merely resides in an decrease of the one digit after the decimal point, when there is a large processing capacity for an industrial plant, the tiny change of the one digit after the decimal point may result in a large variation in the absolute amount of material. It demonstrates that feeding the unreacted diphenylamine during the phenothiazine production process into the intermediate component tower during the diphenylamine production process enables the intermediate component tower of the diphenylamine production process to operate smoothly in the entire cycle; moreover, due to the broadened purity range of the diphenylamine raw material purity used during the phenothiazine production process, the product tower of the diphenylamine synthesis process operates relatively smooth and steady, there is not the circumstance that the diphenylamine product is unqualified after operating for a time period and need to be dumped to the outside world, thereby reducing the energy consumption of the plant and improving the economic efficiency thereof.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

## Claims

1. A method for preparing diphenylamine and phenothiazine from aniline, **characterized in that** the method comprises the following steps:
(1) performing a reaction on an aniline raw material to obtain a reaction mixture containing diphenylamine;
(2) carrying out gas-liquid separation on the reaction mixture obtained in step (1) to obtain a first liquid product and a first gas product;
(3) subjecting the first liquid product to a separation treatment to obtain an aniline reclaimed material and a diphenylamine product;
(4) performing a reaction on a part of the diphenylamine product obtained in step (3) to obtain a reaction mixture containing phenothiazine;
(5) carrying out gas-liquid separation on the reaction mixture obtained in step (4) to obtain a second liquid product and a second gas product, and separating a phenothiazine product from the second liquid product.

2. The method according to claim 1, wherein the catalyst used during the reaction process in step (1) is at least one selected from the group consisting of β molecular sieve catalyst, Y molecular sieve catalyst, X molecular sieve catalyst, mordenite, and ZSM-5.

3. The method according to claim 2, wherein the catalyst used during the reaction process in step (1) is β molecular sieve catalyst;
preferably, the β molecular sieve catalyst comprises 50-95wt% of β molecular sieve and 5-50wt% of γ-Al₂O₃, based on the total weight of the β molecular sieve catalyst, wherein the β molecular sieve has a SiO₂/Al₂O₃ molar ratio within the range of 20-100, preferably within the range of 28-68.

4. The method according to any one of claims 1-3, wherein the reaction in step (1) is a continuous operation or an intermittent operation, preferably a continuous operation.

5. The method according to claim 4, wherein the reaction conditions of the continuous operation in step (1) comprise: a reaction pressure within the range of 1-8MPa, preferably 2-6MPa; a reaction temperature within the range of 250-380°C, preferably 280-360°C; and a liquid hourly volume space velocity within the range of 0.05-1h⁻¹, preferably 0.1-0.5h⁻¹.

6. The method according to any one of claims 1-5, wherein the reaction in step (1) is performed in the presence of a protective gas, which is preferably nitrogen gas and/or hydrogen gas;
preferably, a volume ratio of the protective gas to the aniline raw material is within the range of 10-1,000:1, preferably 50-500:1.

7. The method according to claim 1, wherein the separation treatment process in step (3) is a multi-stage rectification;
preferably, the multi-stage rectification process comprises the following steps:
removing the light component: conveying the first liquid product to a light component removal tower for rectification, and removing the light component from the tower top;
aniline recovery: conveying the tower bottom product of the light component removal tower to an aniline recovery tower for rectification, collecting an aniline reclaimed material from the tower top, and obtaining a diphenylamine-containing material flow from the tower bottom;
removing the intermediate component: feeding the diphenylamine-containing material flow to an intermediate component tower for rectification, removing the intermediate component from the tower top, and obtaining a diphenylamine-rich material flow at the tower bottom;
diphenylamine product rectification: conveying the diphenylamine-rich material flow to a diphenylamine product tower for distillation, receiving the diphenylamine products with different purities from the tower top and side line respectively, and removing the heavy component from the tower bottom.

8. The method according to claim 7, wherein the diphenylamine qualified product is obtained from the tower top of the diphenylamine product tower, the diphenylamine premium grade is extracted from a side line of the diphenylamine product tower, the purity of the diphenylamine qualified product is larger than or equal to 98wt%, the purity of the diphenylamine premium grade is larger than or equal to 99.6wt%; preferably, the diphenylamine qualified product is used as a reaction raw material of step (4).

9. The method according to claim 1, 7 or 8, wherein the aniline reclaimed material obtained in step (3) is recycled as the aniline raw material.

10. The method according to claim 1, wherein the reaction in step (4) is performed in the presence of a sulfur-containing substance, which is at least one selected from the group consisting of sulfur, sodium sulfide, carbon disulfide, and sulfur dioxide.

11. The method according to claim 1 or 10, wherein the catalyst used during the reaction in step (4) is at least one selected from the group consisting of an iodine tablet, anhydrous aluminum trichloride and a solid acid catalyst.

12. The method according to claim 1 or 10, wherein the reaction conditions of the continuous operation in step (4) comprise: a reaction pressure within the range of 5-101kPa, preferably 50-101kPa; a reaction temperature within the range of 130-250°C, preferably 160-220°C; and a liquid hourly volume space velocity within the range of 0.1-2h⁻¹, preferably 0.1-0.5h⁻¹.

13. The method according to claim 1 or 10, the reaction conditions of the intermittent operation in step (4) comprise: a reaction pressure within the range of 5-101kPa, preferably 50-101kPa; a reaction temperature within the range of 130-250°C, preferably 160-220°C; and a reaction time within the range of 2-10h, preferably 4-8h.

14. The method according to claim 1, 10, 11, 12, or 13, wherein the reaction in step (4) is performed in the presence of a protective gas, which is preferably nitrogen gas and/or hydrogen gas;
preferably, the volume ratio of the protective gas to the aniline raw material is within the range of 50-500:1, preferably 10-100:1.

15. The method according to claim 7, wherein the process of separating a phenothiazine product from the second liquid product in step (5) comprises: sequentially subjecting the second liquid product to adsorption purification and vacuum distillation.

16. The method according to claim 15, wherein the diphenylamine-rich light component obtained from the vacuum distillation process is conveyed to the intermediate component tower for rectification.

17. The method according to claim 1, 2, 3, 7, 8, 9, 10, 15, or 16, wherein the first gas product obtained in step (2) and the second gas product obtained in step (5) are mixed for subjecting to the purification treatment.

18. A system for preparing diphenylamine and phenothiazine from aniline, **characterized in that** the system comprises:
a first reactor (100) for performing reaction on an aniline raw material in the first reactor (100);
a first gas-liquid separation device (200) for carrying out gas-liquid separation on the reaction mixture obtained in the first reactor (100) to obtain a first liquid product and a first gas product;
a first product separation device (300) for subjecting the first liquid product separated from the first gas-liquid separation device (200) to a separation treatment to obtain an aniline reclaimed material and a diphenylamine product;
a second reactor (400) for performing a reaction on a part of the diphenylamine product obtained from the first product separation device (300) in the second reactor (400);
a second gas-liquid separation device (500) for carrying out gas-liquid separation on the reaction mixture obtained in the second reactor (400) to obtain a second liquid product and a second gas product; and
a second product separation device (600) for separating a phenothiazine product from the second liquid product separated by the second gas-liquid separation device (500).

19. The system according to claim 18, wherein the first product separation device (300) is a multi-stage rectification device;
preferably, the multi-stage rectification device comprises:
a light component removal tower (310) for rectifying the first liquid product separated by the first gas-liquid separation device (200), and removing the light components from the tower top;
an aniline recovery tower (320) for rectifying the tower bottom product from the light component removal tower (310), collecting an aniline reclaimed material from the tower top, and obtaining a diphenylamine-containing material flow from the tower bottom;
an intermediate component tower (330) for rectifying the diphenylamine-containing material flow from the aniline recovery tower (320), removing the intermediate component from the tower top, and obtaining a diphenylamine-rich material flow at the tower bottom; and
a diphenylamine product tower (340) for rectifying the diphenylamine-rich material flow from the intermediate component tower (330), receiving the diphenylamine products with different purities from the tower top and side line respectively, and removing the heavy component from the tower bottom.

20. The system according to claim 19, wherein a diphenylamine qualified product transfer pipeline (341) for outputting the diphenylamine qualified product is provided at the tower top of the diphenylamine product tower (340); a diphenylamine premium grade collection pipeline (342) for collecting diphenylamine premium grade is provided at the side of the diphenylamine product tower (340); the diphenylamine qualified product transfer pipeline (341) is in communication with the diphenylamine raw material feed inlet of the second reactor (400).

21. The system according to claim 19, wherein the second product separation device (600) comprises:
an adsorption purification device (610) for performing adsorption purification on a second liquid product separated by the second gas-liquid separation device (500); and
a vacuum distillation apparatus (620) for performing vacuum distillation on the liquid product treated by the adsorption purification device (610) to obtain the diphenylamine-rich light component and the phenothiazine product.

22. The system according to claim 21, wherein a diphenylamine-rich material transfer pipeline (621) for outputting the diphenylamine-rich light component is disposed at the top of the vacuum distillation apparatus (620), the diphenylamine-rich material transfer pipeline (621) is in communication with the diphenylamine-containing material flow feed inlet of the intermediate component tower (330).

23. The system according to any one of claims 18-22, wherein the system further comprises a waste gas purification apparatus (800), which is in communication with the first gas product transfer pipeline (201) disposed at the top of the first gas-liquid separation device (200) and the second gas product transfer pipeline (501) disposed at the top of the second gas-liquid separation device (500).
